# EUROPEAN PATENT SPECIFICATION

(11) **EP 2 910 128 B1**
(45) Date of publication and mention of the grant of the patent: **26.09.2018**
(21) Application number: 15155214.8
(22) Date of filing: 16.02.2015
(51) Int. Cl.: A23D 7/005, A21D 2/16, A21D 8/04

(54) **Enzyme-containing solid bakery emulsion**
Enzymhaltige feste Backemulsion
Émulsion de boulangerie solide contenant des enzymes

(30) Priority: 20.02.2014 EP 14156015
(43) Date of publication of application: 26.08.2015
(73) Proprietor: CSM Bakery Solutions Europe Holding B.V., 1019 GM Amsterdam (NL)
(72) Inventor: Demeurisse, Jeroen, 9870 Zulte (BE); Stronati, Raffaele, 20020 Lainate Milan (IT); Tronsmo, Kari Margrete, DE-55411 Bingen am Rhein (DE)
(74) Representative: Nederlandsch Octrooibureau

(56) References cited:
- WO-A1-95/26641
- JP-A- 2003 210 106
- JP-A- 2011 062 086
- US-A- 5 178 897
- US-A1- 2002 094 367

## Description

### TECHNICAL FIELD OF THE INVENTION

The present invention relates to solid bakery emulsions comprising 40-99 wt.% of a continuous fat phase and 1-60 wt.% of a dispersed aqueous phase, said emulsion containing an active enzyme, more particularly a polysaccharide hydrolysing enzyme such as amylase or xylanase.

The solid bakery emulsion according to the present invention can advantageously be used in the preparation of bakery products such as cake and yeast leavened dough. The solid bakery emulsions of the present invention, when intimately mixed with flour and other bakery ingredients to produce a dough or a batter, yield baked products with, for instance, improved fresh keeping properties. The bakery emulsion has excellent shelf stability and the polysaccharide hydrolysing enzyme contained in the emulsion exhibits surprisingly little loss of activity during storage.

Another aspect of the present invention relates to a process of producing the aforementioned solid bakery emulsion.

Yet another aspect of the present invention relates to the use of the solid bakery emulsion in a method of preparing a dough or a batter.

### BACKGROUND OF THE INVENTION

Staling of baked foodstuffs is a well-known problem. Staling, or "going stale", is a chemical and physical process in baked foods that reduces their palatability. Staling becomes evident as an increase of the firmness of the crumb, a decrease of the elasticity or resilience of the crumb, and changes in the crust, which becomes tough and leathery. The increase in crumb firmness, which is considered as the most important aspect of staling, is recognized by the consumer a long time before the bread product has otherwise become unsuitable for consumption.

Staling is not, as is commonly believed, simply a drying-out process due to evaporation of water. Bread will stale even in a moist environment and stales most rapidly at temperatures just above freezing. Although the precise mechanism of staling is still unknown, one important mechanism appears to be migration of moisture from the starch granules into the interstitial spaces, and realigning of amylose and amylopectin molecules of starch. The process of the realignment of the starch molecules is called retrogradation. On retrogradation, crystal-like structures may form that are similar to those originally present in the starch granules, and the process is referred to as recrystalisation. Retrogradation normally results in stale bread with a harder, less resilient crumb texture.

Starch is an essential constituent of baked foodstuffs. During the baking process, the starch becomes gelatinized and absorbs large amounts of water; meanwhile the protein denatures. Immediately after baking, the starch begins to retrograde. The firmness of the crumb increases, although this is still regarded as an advantage in the first hours. In particular the sliceability and chewing characteristics of the crumb improve during this period.

It is assumed that the unbranched starch fraction, amylose, retrogrades first, followed by the branched fraction of the starch, amylopectin, during further storage. At the same time the crumb becomes stiffer, and in the course of time increasingly less elastic and eventually dry and hard: the bread has become stale. In addition, the crust looses crispness and becomes leathery during storage. It is assumed that this is a result of water being released by retrogradation and diffusing outward from the crumb to the crust.

It is undisputed that the causal key reaction for all of these staling phenomena is starch retrogradation. Suppressing or circumventing this phenomenon is the subject matter of numerous protective rights and publications.

One strategy for hindering, at least partly, the considerable firming of the crumb during storage has already been long known: the crumb is made softer from the beginning. The means of choice to do this are emulsifiers such as mono/diglycerides, which are added to the dough and produce a crumb structure that is particularly soft from the beginning.

The use of α-amylase derived from fungi such as *Aspergillus oryzae* has a similar effect. It acts upon damaged starch particles, thereby lowering the viscosity of the dough and producing fermentable sugars. As a consequence, the finished baked article has larger volume, which is consistent with softer crumb. Aside from the fact that the fresh bread is soft, this strategy does not prevent or inadequately prevents the development of a harder, less elastic consistency of the crumb when it becomes stale.

A further strategy is to reduce retrogradation by enzyme-mediated partial hydrolysis of the two starch fractions during baking. Enzyme-mediated hydrolysis of the crumb should preferably take place after the starch has been gelatinized, i.e., above about 65°C. As a consequence the structure of the starch in the baked product is radically altered, limiting its ability to retrograde. Heat-stable maltogenic amylases are widely used in the baking industry as an anti-staling agent due to their ability to reduce retrogradation by hydrolyzing starch at starch gelatinization temperature.

Anti-staling agents such as mono/diglycerides and enzymes are usually added during dough or batter preparation as a single ingredient or as part of bakery mix (e.g. a cake mix or a bread improver).

Bakery products containing a combination of fat and enzyme have been described in the prior art.

EP-A 0 572 051, for instance, describes a liquid bread-improver composition comprising 75 - 95 wt. % of a vegetable oil, 1 - 5 wt. % of a hydrogenated vegetable oil, 1-5 wt. % of a partly hydrogenated vegetable oil and emulsifiers, flavours, bread-improving enzymes and oxidants.

WO 2003/039261 describes a water-free low viscous liquid bread improver comprising a liquid emulsifier based on esters of hydroxy polycarboxylic acid derivatives, a glyceride based stabilizer, additive(s) and less than about 20 % oil. In the examples, enzymes are used as additives.

US 4,614,656 describes a baking composition wherein margarine is segregated from other baking ingredients in a composite product comprising margarine and a water-free fatty phase. The water-free fatty phase comprises baking ingredients dispersed therein. Example 2 describes a baking composition with a water-free fatty phase containing dispersed enzyme.

Water-in-oil emulsions containing enzymes have also been described in the prior art.

US 4,961,939 describes a water-in-oil emulsion having an oil phase comprising fish oil and a water or milk phase. The water or milk phase comprises a stabilizer containing enzymes, which are present in such amounts as to prevent or retard the formation of malodorous alcohols and/ or aldehydes in fish oil during the shelf life of such emulsion. The stabilizer containing enzymes are selected from
a) aldehyde dehydrogenase plus alcohol dehydrogenase plus nicotinamide adenine dinucleotide,
b) Aldehyde oxidase plus alcohol oxidase plus catalase, or
c) Xanthine oxidase plus alcohol oxidase plus catalase.

WO 2009/046988 describes a water-in-oil emulsion comprising an oil, water, a phospholipase and a phosphatide for the use in laminated doughs.

EP-A 1 982 597 describes water-continuous edible fat replacer compositions comprising water and a mixture of mono- and diglycerides, both components representing more than 88% by weight and less than 100% by weight, based on the total weight of said fat replacer composition, said mixture of mono- and diglycerides being in an amount comprised between 8 wt% and 60 wt%, said composition further comprising at least one gum for increasing the viscosity in an amount sufficient for reaching 100% by weight. Example 1 describes such edible fat replacer composition containing enzyme.

JP 2011/062086 describes margarines containing α-amylase, maltogenic α-amylase or glucoamylase and the use of these margarines in the preparation of bread.

JP 2003/210106 describes a method of preparing a water-in-oil emulsion containing an emulsifier and α-amylase.

### SUMMARY OF THE INVENTION

The inventors have developed a solid bakery emulsion that can advantageously be used in the preparation of baked products with improved fresh keeping and textural properties. The bakery emulsion has excellent shelf stability and during its shelf-life the emulsion's performance remains constant.

Examples of baked products that can suitably be prepared by intimately mixing the solid bakery emulsion of the present invention with flour and other bakery ingredients include cake and yeast leavened dough (e.g. dough for buns or brioche).

The solid bakery emulsion of the present invention comprises: 40-99 wt.% of a continuous fat phase having a solid fat content at 20°C (N₂₀) of at least 12% and 1-60 wt.% of a dispersed aqueous phase, said dispersed aqueous phase having a pH in the range of 3.2 to 5.5, and said emulsion containing an active polysaccharide hydrolysing enzyme selected from amylase, xylanase and combinations thereof.

The solid bakery emulsion of the present invention is ideally suited for use in the preparation of dough or batter. The emulsion can easily be mixed with flour and other ingredients to produce a homogeneous dough or batter. During such mixing, the solid bakery emulsion is evenly distributed throughout the dough or batter.

Although the inventors do not wish to be bound by theory, it is believed that the even distribution of the emulsion through the dough or batter brings the enzyme component into intimate contact with e.g. the starch or arabinoxylans naturally present in the flour when it is released from the emulsion, thereby allowing the enzyme to rapidly hydrolyse such polysaccharide substrate. In comparison to baked products prepared with a conventional bakery emulsion, baked products prepared with the present solid bakery emulsion tend to have a softer crumb, a more cohesive crumb and/or improved resistance to staling in case the enzyme used is amylase. In case the enzyme employed is xylanase, a less sticky dough is obtained and the baked product tends to have a higher specific volume and improved crumb structure.

The inclusion of enzyme in the bakery emulsion of the present invention offers the advantage that interaction between enzyme and flour components can be delayed. By manipulating the fat phase and/or aqueous phase of the bakery emulsion, e.g by employing a high melting continuous fat phase or a gelled aqueous phase, it can be ensured that a substantial part of the enzyme activity present in the emulsion is released during proofing (at elevated temperature) and/or during heating (e.g. baking) of the dough or batter in which the emulsion is applied. Thus, adverse impact of enzyme activity on dough handling properties may be minimized effectively.

The bakery emulsion of the present invention offers the important advantage that it combines excellent shelf-stability with no more than limited loss of enzyme activity during its shelf-life.

Another aspect of the invention relates to a process of producing the solid bakery emulsion of the present invention, comprising:
- mixing an aqueous phase composition and a fat phase composition to prepare a pre-emulsion, said pre-emulsion having a temperature in the range of 40-85 °C; and
- cooling the pre-emulsion;
wherein the polysaccharide hydrolysing enzyme is contained in the aqueous phase composition and/or the fat phase composition.

The invention further provides a method of preparing a dough or batter, said method comprising thoroughly mixing flour and other bakery ingredients with the solid bakery emulsion of the present invention.

### DETAILED DESCRIPTION OF THE INVENTION

Accordingly, one aspect of the invention relates to a solid bakery emulsion comprising 40-99 wt.% of a continuous fat phase and 1-60 wt.% of a dispersed aqueous phase, said continuous fat phase having a solid fat content at 20°C (N₂₀) of at least 12%, said dispersed aqueous phase having a pH in the range of 3.2 to 5.5, and said emulsion containing an active polysaccharide hydrolysing enzyme selected from amylase, xylanase and combinations thereof.

The terms "wt.%" and "% by weight" refer to the concentration expressed on a weight-by-weight basis (% (w/w)).

The term "fat" or "oil" as used herein, unless indicated otherwise, refers to lipids selected from triglycerides, diglycerides, monoglycerides, fatty acids, phosphoglycerides and combinations thereof. Unless indicated otherwise, the fat and oil present in the solid bakery emulsion is deemed to be part of the continuous fat phase.

The term "polysaccharide" as used herein refers to a polymer comprising at least 10 monosaccharide units linked to one another by glycosidic bonds.

The term "starch" as used herein refers to a polysaccharide consisting of a large number of glucose units joined by glycosidic bonds. Naturally occurring starch consists of two types of molecules: the linear polysaccharide amylose and the branched polysaccharide amylopectin.

The term "amylase" as used herein refers to an enzyme that is capable of catalyzing the hydrolysis of starch.

The term "a-amylase" or "1,4-α-D-glucan glucanohydrolase" as used herein refers to endo-acting hydro lases (EC class 3.2.1.1) that are capable of cleaving the α-D-(1→4) - glycosidic linkages in starch. The endo-acting activity of α-amylase allows the enzyme to cleave α-D-(1→4) -glycosidic linkages within the starch molecule in a random fashion yielding polysaccharides containing three or more (1-4)-α-linked D-glucose units.

The term "maltogenic α-amylase" or "glucan 1,4-α-maltohydrolase" as used herein refers to exo-acting amyloytic enzymes (EC class 3.2.1.133) that, starting from the non-reducing end of the substrate, is capable of catalyzing hydrolysis of the 1,4-α-D-glucosidic linkages in polysaccharides in order to remove successive α-maltose residues from the non-reducing end.

The term G4 amylase refers to 1,4- α-D-glucan maltotetrahydrolase (EC class 3.2.1.60).

The term "amyloglucosidase" as used herein refers to another starch degrading enzyme (EC class 3.2.1.3). Amyloglucosidase (or 1,4- α-D-glucan glucohydrolase) not only cleaves the last a(1,4)glycosidic linkages at the non-reducing end of amylose and amylopectin, yielding glucose, but also cleaves a(1,6)glycosidic linkages.

The term "xylanase" or "endo-1,4-beta (β)-xylanase." as used herein refers to a class of endo-acting enzymes (EC class 3.2.1.8) that are capable of hydrolysing the 1,4-β-D-xylosidic linkages in xylans.

The solid fat profile of the fat phase is determined by measuring the N-value at different temperatures. The N value at temperature x °C is referred to in here as Nₓ and represents the amount of solid fat at a temperature of x °C. These N-values can suitably be measured using the generally accepted analytical method that is based on NMR measurements (AOCS official method Cd 16b-93): Sample pre-treatment involves heating to 80°C 15 minutes, 15 minutes at 60°C, 60 minutes at 0°C and 30 minutes at the measuring temperature.

The polysaccharide hydrolysing enzyme employed in the present bakery emulsion preferably is heat stable. The use of a highly heat stable enzyme offers the advantage that heat employed during the manufacturing process to melt ingredients and/or to reduce microbial count have limited effect on the activity of the enzyme. The polysaccharide hydrolysing enzyme is considered to be heat stable if it retains (at least 50% of) its activity when a dilute aqueous solution of the enzyme has been heated to 70°C for 1 minute.

In accordance with another preferred embodiment, the polysaccharide hydrolysing enzyme is sugar tolerant. The use of sugar tolerant enzyme offers the advantage that the bakery emulsion may suitably be used in the preparation of sweetened dough (e.g. brioche dough) or sweetened batter (e.g. cake batter). The polysaccharide hydrolysing enzyme is considered to be sugar tolerant if it retains at least 50% of its activity in baking recipes (dough or batter) containing 5% sucrose.

The amylase employed in the bakery emulsion is preferably selected from α-amylase, maltogenic α-amylase, G4 amylase, amyloglucosidase and combinations thereof. More preferably, the amylase is selected from α-amylase, maltogenic α-amylase and combinations thereof. The amylase employed in the bakery emulsion is most preferably maltogenic α-amylase. Examples of commercially available maltogenic α-amylases that may be incorporated in the bakery emulsion of the present invention include Opticake® and Novamyl®.

In accordance with one preferred embodiment, the bakery emulsion of the present invention contains maltogenic amylase in a concentration of 3-2400 MAU/g, more preferably of 4-2000 MAU/g, most preferably of 10-500 MAU/g.

One maltogenic amylase unit (MAU) is defined as the amount of enzyme required to release one nanomol of maltose per second at a concentration of 20 mg of maltotriose substrate per ml of 0.05 M citrate buffer, pH 5.0 at 37°C.

According to another preferred embodiment, the bakery emulsion contains α-amylase in a concentration of 2-1300 AU/g, more preferably of 3-1050 AU/g and most preferably of 10-500 AU/g.

One α-amylase unit (AU) is defined as the amount of enzyme required to release one nanomol of reducing sugar per second at a concentration of 10 mg of starch substrate per ml of 0.05 M MES6+ buffer (50 mM 2-morpholinoethanesulfonic acid + 20 g/l calcium acetate + 2 g/l sodium chloride), pH 6 at 50°C.

According to yet another preferred embodiment, the emulsion contains amyloglucosidase in a concentration of 0.2-100 AGU/g, more preferably of 1-60 AGU/g.

One unit of amyloglucosidase activity (AGU) is defined as the amount of enzyme required to release one nanomol of glucose per second at a concentration of 20 mg of maltose substrate per ml of 0.05 M citrate buffer, pH 5.0 at 37°C.

According to another preferred embodiment, the bakery emulson contains xylanase in a concentration of 0.2-100 XU/g, more preferably 1-60 XU/g and most preferably 5-50 XU/g.

One xylanase unit (XU) is defined as the amount of enzyme required to release one nanomol of xylose per second at a concentration of 10 mg of xylan substrate per ml of 0.05 M citrate buffer, pH 5.3 at 37°C.

The active polysaccharide hydrolysing enzyme may be contained in the dispersed aqueous phase of the bakery emulsion, the continuous fat phase of the emulsion or both. Typically, at least 50 wt.%, more preferably at least 80 wt.% and most preferably at least 90 wt.% of said active enzyme is contained in the aqueous phase.

As mentioned before, the bakery emulsion of the present invention offers the important advantage that only limited loss of enzyme activity is observed during normal storage of the emulsion. This is surprising because enzymes tend to rapidly lose activity when dissolved in water, especially if the water contains other components that adversely affect enzyme activity. This is why commercially available bakery products containing fat and enzymes have been formulated as water-free products.

The aqueous phase of the present emulsion contains nutrients (e.g.enzyme and the carrier material used in the enzyme preparation) that can be digested by micro-organisms. Consequently, the aqueous phase needs to be preserved in order to prevent microbial growth. However, conventional measures for preventing microbial growth, i.e. heat treatment, pH reduction, salt addition, preservatives can have a pronounced adverse impact on enzyme activity.

The inventors have been able to provide a bakery emulsion with an aqueous phase that is microbiologically stable and in which the activity of the enzyme is surprisingly stable over time. This is achieved by adjusting the pH of the aqueous phase to a pH in the range of 3.2 to 5.5 with the help of a suitable acidulant.

According to a particularly preferred embodiment, the aqueous phase of the solid bakery emulsion of the present invention has a pH in the range of 3.4 to 5.3, more preferably of 3.5 to 5.1 and most preferably of 3.6 to 5.0.

The bakery emulsion preferably contains 0.01 wt.% of an organic acid selected from lactic acid, citric acid, tartaric acid, acetic acid and combinations thereof, more preferably selected from lactic acid, citric acid, tartaric acid and combinations thereof. Most preferably, the organic acid is lactic acid.

Minor quantities of preservatives may also suitably be applied in the aqueous phase of the present emulsion to achieve optimum microbial stability without significant reduction of enzyme activity. In particular if these preservatives are applied in an aqueous phase with a mildly acidic pH an optimum balance between microbial safety and enzyme activity can be achieved.

Accordingly, the aqueous phase of the solid bakery emulsion of the present invention typically contains at least 0.02 wt.%, more preferably at least 0.03 wt.%, even more preferably at least 0.05 wt.% and most preferably at least 0.05 wt.% of a preservative selected from sorbate, benzoate, propionate and combinations thereof. Such preservative is preferably present in the aqueous phase in a concentration that does not exceed 2 wt.%, more preferably does not exceed 1 wt.% and most preferably does not exceed 0.5 wt.%. Most preferably, the preservative used is sorbate. Typically, the aqueous phase contains 0.02-1.5 wt.%, more preferably 0.05-0.8 wt.% of sorbate.

The application of salt in the aqueous phase may also help to prevent microbial growth. In accordance with a preferred embodiment, salt is employed in combination with preservative and/or acidulant to achieve maximum preservative action with minimum impact on enzyme activity.

Accordingly, the aqueous phase of the solid bakery emulsion of the present invention typically contains less than 100 mg/ml of sodium. Preferably, the aqueous phase contains 2-50 mg/ml of sodium.

The solid bakery emulsion of the present invention typically has a solid fat content at 20°C that is significantly higher than that of a table spread and considerably lower than that of a conventional puff pastry margarine. Accordingly, N₂₀ of the solid bakery emulsion preferably is in the range of 15-35%, more preferably in the range of 17-30%.

The solid bakery emulsion of the present invention typically has a solid fat content at at 25°C (N₂₅) of 8-30%, a solid fat content at 35°C (N₃₅) of less than 15% and a solid fat content at 40°C (N₄₀) of not more than 10%.

The solid bakery emulsion of the present invention contains a continuous fat phase that exhibits a considerable drop in solid fat within the temperature range of 20-30 °C. Thus, in accordance with a preferred embodiment, N₂₀-N₃₀ is in the range of 6-25%, more preferably in the range of 9-20%.

Likewise, N₂₀-N₄₀ preferably is within the range of 10-35%, more preferably of 12-30%.

The ratio (N₂₀-N₄₀)/N₂₀ is indicative of the rate at which the continuous fat phase melts within the temperature range of 20 to 40°C. Preferably, the continuous fat phase of the emulsion shows considerable melting within this temperature range. Accordingly, in accordance with a preferred embodiment (N₂₀-N₄₀)/N₂₀ is at least 0.7, more preferably at least 0.75.

Triglycerides typically represent the bulk of the continuous fat phase. Typically, triglycerides represent at least 80 wt.%, more preferably at least 90 wt.% of the continuous fat phase.

The bakery emulsion of the present invention typically is sufficiently hard to be packaged in a wrapper. The so called Stevens value is a suitable measure of the hardness of the present emulsion. The Stevens value at 20°C (S₂₀) is determined according to the following protocol. The temperature of the sample is maintained at 20°C for at least 24 hours before measuring the hardness of the sample at 20°C with a TA.XTplus Texture Analyser from Stable Microsystems equipped with a stainless steel probe with a diameter of 4.4 mm The probe is pushed into the product at a speed of 2 mm/s over a distance of 10 mm, using a trigger force of 5 gram. The maximum force required is expressed in grams.

The solid bakery emulsion of the present invention typically has a S₂₀ of at least 100 g, more preferably of 100-250 g and most preferably of 150-200g.

The solid bakery emulsion typically contains 60-97 wt.% of a continuous fat phase and 3-40 wt.% of a dispersed aqueous phase. More preferably, the emulsion contains 65-95 wt.% of a continuous fat phase and 5-35 wt.% of a dispersed aqueous phase. Most preferably, the solid bakery emulsion contains 70-90 wt.% of a continuous fat phase and 10-30 wt.% of a dispersed aqueous phase.

Together, the continuous fat phase and the dispersed aqueous phase typically constitute at least 70 wt.%, more preferably at least 85 wt.% and most preferably at least 95 wt.% of the solid bakery emulsion.

The aqueous phase of the solid bakery emulsion may suitably contain edible ingredients such as salt, hydrocolloids, non-hydrocolloid protein, dispersed fat, flavouring, acidulant and preservatives. An example of a non-hydrocolloid protein is milk protein.

The fat phase of the solid bakery emulsion typically is composed of a blend of fats and oils. Examples of fats and oils that may be employed include highly unsaturated liquid oils (e.g. sunflower oil, soybean oil and/or rapeseed oil), lauric fats (e.g. coconut oil and/or palm kernel oil), palm oil and milk fat. Also olein and/or stearin fractions of these oils and fats may suitably be used. Likewise, the aforementioned oils or blends of these oils and fats may be employed in hydrogenated and/or interesterified form.

The fat phase of the emulsion may also contain other edible ingredients besides fat. Examples of such edible ingredients include colouring, flavouring and anti-oxidants.

Another aspect of the invention relates to a method of preparing a dough or batter comprising thoroughly mixing flour with a solid bakery emulsion as defined herein before.

The dough or batter obtained by the present method is suitably divided in portions that are cooked to prepare a ready-to-eat products. Cooking methods that may be employed to prepare such ready-to-eat products include baking, frying and boiling. Most preferably, the portions of dough or batter are cooked by baking. Typically, during cooking, the core temperature of the portions of dough of batter is increased to at least 70°C, more preferably at least 90°C.

The bakery emulsion is typically employed in the present method in a concentration of 5-110% by weight of flour, more preferably in a concentration of 10-100 % by weight of flour.

In accordance with a particularly preferred embodiment, the bakery emulsion contains maltogenic α-amylase and is employed in the aforementioned method in an amount that provides 0.2-240 MAU per gram of flour, more preferably 0.5-150 MAU per gram of flour and most preferably 1-40 MAU per gram of flour.

In accordance with another preferred embodiment, the bakery emulsion contains α-amylase and is employed in an amount that provides 2-200 AU per gram of flour, more preferably 6-140 AU per gram of flour.

In accordance with yet another preferred embodiment, the present emulsion contains xylanase and is employed applied in an amount that provides 0.1-20 XU per gram of flour, more preferably 0.3-8 XU per gram of flour.

In accordance with one advantageous embodiment of the invention, the method is used to prepare a yeast-leavened dough, said method comprising thoroughly mixing flour with the bakery emulsion, active yeast, water and optionally further bakery ingredients. Examples of yeast-leavened dough that can suitably be produced in this fashion include bun dough and brioche dough.

Typically, the yeast-leavened dough is prepared by employing the bakery emulsion in a concentration of 5-60% by weight of flour, more preferably of 10-50% by weight of flour.

The water content of the yeast-leavened dough typically lies in the range of 40-60% by weight of flour. More preferably, the water content is within the range of 45-55% by weight of flour.

In another preferred embodiment, the method is used to prepare batter, said method comprising thoroughly mixing flour with the bakery emulsion, sugar, eggs and further bakery ingredients. Examples of batter that may suitably be prepared by this method include cake batter and muffin batter

The batter is typically prepared by employing the bakery emulsion in a concentration of 10-100% by weight of flour, more preferably of 30-100% by weight of flour. Sugar is typically employed in the batter in a concentration of 50-150% by weight of flour. Egg solids are preferably employed in a concentration of 25-100% by weight of flour.

The water content of the batter preferably lies in the range of 20-100% by weight of flour, more preferably in the range of 25-80% by weight of flour.

A further aspect of the invention relates to a process of producing the solid bakery emulsion of the present invention, said process comprising preparing an aqueous phase composition containing the polysaccharide hydrolysing enzyme and maintaining the temperature of the aqueous phase composition below 85°C; preparing a fat phase composition; mixing the aqueous phase composition and the fat phase composition to prepare a pre-emulsion, said pre-emulsion having a temperature in the range of 40-85 °C; and cooling the pre-emulsion.

The process of producing the solid bakery emulsion of the present invention alternatively comprises preparing an aqueous phase composition; preparing a fat phase composition containing the polysaccharide hydrolysing enzyme and maintaining the temperature of the fat phase composition below 85°C; mixing the aqueous phase composition and the fat phase composition to prepare a pre-emulsion, said pre-emulsion having a temperature in the range of 40-85 °C; and cooling the pre-emulsion.

The pre-emulsion may suitably be cooled by passing the pre-emulsion through a heat exchanger. According to a preferred embodiment, the pre-emulsion is subjected to mechanical working during the cooling so as to ensure that an emulsion with plastic (as opposed to brittle) consistency is obtained. Scraped surface heat exchangers are an example of a type of heat exchanger that can be used to cool the pre-emulsion. Typically, the pre-emulsion is cooled in the heat exchanger to a temperature of less than 20°C, more preferably of less than 15°C.

Before the pre-emulsion is cooled it is preferably kept at a temperature of at least 50°C, more preferably of at least 55°C and most preferably of at least 60°C for at least 15 seconds. It is preferred to heat the pre-emulsion in this manner to ensure that all fat crystals have melted. It is important to ensure that the fat present in the pre-emulsion is fully melted before the cooling, as fat crystals in the pre-emulsion influence fat crystallization during the cooling process, making it more difficult to control the properties of the final product.

The conditions employed in the present process are preferably such that the enzyme is not exposed to temperatures in excess of 80°C for more than 1 minute.

The invention is further illustrated by the following non-limiting examples.

### EXAMPLES

### Example 1

Starting from the recipe shown in Table 1, three bakery emulsions and one bakery shortening were produced as follows:
- The fat blends were molten by heating to 50°C in a premix vessel.
- The emulsifier (Myverol 18-04K) was combined with part of the fat blend and dissolved therein by heating the blend to a temperature above the melting point of the emulsifier (min. 66°C). After melting the dissolved emulsifier was added to the fat blend. Next, if required, the enzyme (Opticake 50BG) was dispersed in the molten fat blend (temperature 50°C).
- The aqueous phase was prepared by successively dissolving potassium sorbate, lactic acid and - if required - Opticake 50 BG into water at a temperature of 40°C. The pH of the aqueous phase was 4.0.
- The aqueous phase was added to the fat phase under vigorous stirring to obtain a fat continuous emulsion. The temperature of the emulsion was kept at maximum 45°C.
- The emulsion was heated in a plate heat exchanger (65°C / 45 seconds / intended to erase crystal memory) before being fed to a series of scraped surface heat exchangers (A-units), a crystallising unit (C-unit) and a B-unit.
- The crystallised bakery emulsion so produced was packaged in a wrapper.

Emulsion A and Shortening A are comparative examples.

**Table 1**

| | **Emulsion 1** | **Emulsion 2** | **Emulsion A** | **Shortening A** |
|---|---|---|---|---|
| **Fat phase** | **80.2%** | **80,2%** | **80,2%** | **100.0%** |
| *Rapeseed oil* | 20.75% | 20.79% | 20.79% | 25.85% |
| *Palm oil* | 22.34% | 22.39% | 22.39% | 27.84% |
| *Interesterified blend* | 29.52% | 29.58% | 29.58% | 36.79% |
| *Palm fraction* | 7.18% | 7.20% | 7.20% | 8.95% |
| *Myverol 18-04¹* | 0.25% | 0.25% | 0.25% | 0.25% |
| *Opticake 50 BG ²* | 0.16% | 0.00% | 0.00% | 0.20% |

| **Water phase** | **19.8%** | **19.8%** | **19.8%** | **0.0%** |
|---|---|---|---|---|
| *Water* | 19.71% | 19.55% | 19.71% | 0.00% |
| *Potassium sorbate* | 0.04% | 0.04% | 0.04% | 0.00% |
| *Lactic acid (80%)* | 0.05% | 0.05% | 0.05% | 0.00% |
| *Opticake 50 BG ²* | 0.00% | 0.16% | 0.00% | 0.00% |
| | **100%** | **100%** | **100%** | **100%** |

| | | | | |
|---|---|---|---|---|
| ¹ ex Kerry Ingredients, distilled monoglycerides, min. 95% monoglycerides, Iodine Value max. 3, source: palm ² ex Novozymes, enzyme preparation contains maltogenic alpha-amylase (40 MAU/mg) | | | | |

The N-line characteristics of the fat phases and the Stevens hardness of the emulsions at 20 °C are shown in Table 2.

**Table 2**

| | **Emulsion 1** | **Emulsion 2** | **Emulsion A** | **Shortening A** |
|---|---|---|---|---|
| *N10* | 47% | 47% | 47% | 47% |
| *N15* | 37% | 37% | 37% | 37% |
| *N20* | 27% | 27% | 27% | 27% |
| *N25* | 19% | 19% | 19% | 19% |
| *N30* | 11% | 11% | 11% | 11% |
| *N35* | 6% | 6% | 6% | 6% |
| *S20* | 158 g | 200 g | 172 g | 160 g |

### Example 2

Yeast dough (brioche) was prepared on the basis of the recipe shown in Table 3, using Bakery Emulsions 1, 2 and A of Example 1.

**Table 3**

| **Ingredient** | **Weight (g)** | **Temperature (°C)** |
|---|---|---|
| Flour | 2,000 | 20 |
| Sugar | 200 | 20 |
| Salt | 30 | 20 |
| Water | 1,000 | 5 |
| Yeast | 160 | 5 |
| Bakery emulsion | 250 | 20 |

Yeast dough (brioche) was also prepared on the basis of the recipe shown in Table 4, using Shortening A of example 1.

**Table 4**

| **Ingredient** | **Weight (g)** | **Temperature (°C)** |
|---|---|---|
| Flour | 2,000 | 20 |
| Sugar | 200 | 20 |
| Salt | 30 | 20 |
| Water | 1,000 | 5 |
| Yeast | 160 | 5 |
| Shortening | 200 | 20 |
| Acidified water¹ | 50 | 20 |

| | | |
|---|---|---|
| ¹ 99.55% water / 0.25 % lactic acid (80% solution in water) / 0.2% potassium sorbate | | |

The yeast dough was prepared as follows:
- All ingredients were kneaded in a Diosna Spiral kneader for 2 minutes at speed 1 and 3.5 minutes at speed 2. The temperature of the obtained dough was 25-26°C.
- The dough was rounded and given a resting time of 15 minutes
- The dough was divided in pieces of 600 g, the dough pieces were rounded and given a resting time of 5-10 minutes
- The dough pieces were rolled out, using a lamination table, to a final thickness of 3,5 mm in 6 steps (15mm-12.8mm-10.6mm-7mm-4.5mm-3.5mm)
- The rolled-out dough was rolled manually into a bread-like shape and placed in a greased baking tin (8 cm x 9 cm x 31 cm)
- Proofing: 40 minutes at 37°C (humidity 85%)
- Baking: 45 minutes at 200°C in deck oven

The properties of the yeast doughs and baked products obtained using emulsions and shortening 2 weeks after production are described in Table 5.

**Table 5**

| | **Emulsion 1** | **Emulsion 2** | **Emulsion A** | **Shortening A** |
|---|---|---|---|---|
| Specific volume (ml/g) | 3.84 | 3.73 | 3.69 | 3.64 |
| Mouthfeel score¹ after 1 day | 5 | 5 | 3 | 3 |
| Crumb hardness² | | | | |
| After 1 day | 519 ± 36 | 457 ± 29 | 744 ± 29 | 701 ± 39 |
| After 2 days | 565 ± 81 | 508 ± 51 | 1051 ± 63 | 852 ± 57 |
| After 3 days | | | | 865 ± 60 |
| After 4 days | 504 ± 44 | 566 ± 59 | 1083 ± 107 | |
| After 6 days | | | | 1042 ± 135 |
| After 7 days | 641 ± 114 | 633 ± 84 | 1357 ± 165 | |

| | | | | |
|---|---|---|---|---|
| ¹ Mouthfeel score: score between 1 (bad) and 5 (very good) ² Crumb hardness: crumb sample to be analysed is prepared by cutting pieces (50x50x25mm) from the crumb. To measure the crumb hardness, a Texture Profile Analysis (TPA) is performed using a TA.XT₂ from Stable Micro Systems. Following settings were programmed: - Load cell: 5kg - Probe: 50mm ø Cylinder Aluminium Probe - P/50 - TA.XT₂-settings   ▪ Pretest speed: 1mm/s   ▪ Test speed: 5mm/s   ▪ Posttest speed: 5mm/s   ▪ Compression depth: 10mm (40% compression on 25mm sample)   ▪ Time between cycles: 5s   ▪ Trigger type: automatic on 5g   ▪ Data acquisition rate: 200pps | | | | |

The crumb hardness is the first positive peak in the graph, meaning the maximum force expressed in grams needed to compress the sample during the first cycle. Indicated results are average values ± standard deviation from 3 pieces per crumb.

The properties of the baked products obtained using emulsions and shortening 8 weeks and 12 weeks after production are described in Table 6 and Table 7, respectively.

**Table 6**

| **Crumb hardness** | **Emulsion 1** | **Emulsion 2** | **Emulsion A** | **Shortening A** |
|---|---|---|---|---|
| After 1 day | 456 ± 12 | 469 ± 59 | 710 ± 77 | 594 ± 34 |
| After 2 days | 519 ± 34 | 514 ± 64 | 902 ± 55 | 693 ± 56 |
| After 3 days | | | | 856 ± 28 |
| After 4 days | 553 ± 52 | 487 ± 54 | 1245 ± 97 | |
| After 6 days | | | | 1193 ± 80 |
| After 7 days | 681 ± 70 | 681 ± 92 | 1394 ± 170 | |
| After 10 days | | | | 2057 ± 163 |
| After 11 days | 887 ± 80 | 611 ± 106 | 3023 ± 703 | |
| After 14 days | 1038 ± 71 | 1207 ± 267 | 4214 ± 332 | 4276 ± 217 |

**Table 7**

| **Crumb hardness** | **Emulsion 1** | **Emulsion 2** | **Emulsion A** | **Shortening A** |
|---|---|---|---|---|
| After 2 days | 526 ± 10 | 600 ± 62 | 948 ± 7 | 980 ± 53 |
| After 6 days | | | | 1589 ± 153 |
| After 7 days | 750 ± 49 | 734 ± 34 | 1855 ± 55 | |
| After 10 days | | | | 3524 ± 959 |
| After 11 days | 870 ± 70 | 1060 ± 156 | 3754 ± 426 | |

### Example 3

Starting from the recipes shown in Table 8, four bakery emulsions were produced (not according to the invention).

**Table 8**

| | **Emulsion 1** | **Emulsion 2** | **Emulsion 3** | **Emulsion 4** |
|---|---|---|---|---|
| **Fat phase** | **80%** | **80%** | **80%** | **80%** |
| *Rapeseed oil* | 20.8% | 20.8% | 20.8% | 20.8% |
| *Palm oil* | 22.4% | 22.4% | 22.4% | 22.4% |
| *Palm fraction* | 7.2% | 7.2% | 7.2% | 7.2% |
| *Interesterified blend* | 29.4% | 29.4% | 29.4% | 29.4% |
| *Myverol 18-04K* | 0.2% | 0.2% | 0.2% | 0.2% |
| *Opticake 50 BG* | 0.04% | 0.00% | 0.00% | 0.04% |

| **Water phase** | **20%** | **20%** | **20%** | **20%** |
|---|---|---|---|---|
| *Water* | 19% | 19.96% | 18.96% | 20% |
| *Salt* | 1% | 0% | 1% | 0% |
| *Opticake 50 BG* | 0.00% | 0.04% | 0.04% | 0.00% |

Emulsions 1 and 4 were batch-wise produced using a lab scale margarine processing unit as follows:
- The fat blends of emulsions 1 and 4 were molten by heating to respectively 50°C and 85°C in a premix vessel (start recording temperature in premix vessel, see Table 10). The enzyme was dispersed into the fat phase.
- After 5 minutes, the aqueous phase (35°C) was added to the fat phase under vigorous stirring to obtain a fat continuous emulsion. The emulsion temperature of emulsions 1 and 4 dropped to 43.3°C and 63.8°C, respectively (Table 10).
- The temperature in the premix vessel was measured at regular intervals (Table 10). The total processing time was 40 minutes. First margarine samples were taken after 15 minutes processing time.

Emulsions 2 and 3 were batch-wise produced using a lab scale margarine processing unit as follows:
- The fat blends of emulsions 2 and 3 were molten by heating to respectively 59°C and 111°C in a premix vessel.
- The aqueous phase (35°C, containing the enzyme) was immediately added to the fat phase under vigorous stirring to obtain a fat continuous emulsion. The emulsion temperature of emulsions 2 and 3 immediately dropped to 50°C and 85°C, respectively (Table 10).
- The temperature in the premix vessel was measured at regular intervals (Table 10). The total processing time was 40 minutes. First margarine samples were taken after 15 minutes processing time.

The N-line characteristics of the fat phases and the Stevens hardness of the emulsions at 20°C are shown in Table 9.

**Table 9**

| | **Emulsion 1** | **Emulsion 2** | **Emulsion 3** | **Emulsion 4** |
|---|---|---|---|---|
| *N10* | 45.3% | 45.3% | 45.3% | 45.3% |
| *N15* | 34.7% | 34.7% | 34.7% | 34.7% |
| *N20* | 25.2% | 25.2% | 25.2% | 25.2% |
| *N25* | 17.9% | 17.9% | 17.9% | 17.9% |
| *N30* | 10.5% | 10.5% | 10.5% | 10.5% |
| *N35* | 5.6% | 5.6% | 5.6% | 5.6% |
| | | | | |
| *S20* | 209 g | 288 g | 267 g | 192 g |

The recorded temperatures in the premix vessel are shown in Table 10.

**Table 10**

| **Emulsion 1** | | **Emulsion 2** | | **Emulsion 3** | | **Emulsion 4** | |
|---|---|---|---|---|---|---|---|
| Time (min) | Temp. (°C) | Time (min) | Temp. (°C) | Time (min) | Temp. (°C) | Time (min) | Temp. (°C) |
| 0 | 50 | 0 | 50 | 0 | 85 | 0 | 85 |
| 5 | 48 | 5 | 49.6 | 5 | 79.6 | 5 | 77 |
| 6 | 43.3 | 14 | 48.5 | 14 | 72.2 | 6 | 63.8 |
| 11 | 48 | 23 | 48.5 | 28 | 64.8 | 15 | 63 |
| 16 | 48.3 | 28 | 48 | 38 | 62.9 | 25 | 62.2 |
| 40 | 48.7 | 40 | 48 | 40 | 62.9 | 40 | 61.5 |

Yeast doughs (brioche) were prepared two weeks after production of the bakery emulsions on the basis of the recipe and processing described in Example 2. The characteristics of the baked products so obtained are shown in Table 11.

**Table 11**

| | **Emulsion 1** | **Emulsion 2** | **Emulsion 3** | **Emulsion 4** |
|---|---|---|---|---|
| *Specific volume (ml*/*g)* | 4.17 | 4.31 | 4.03 | 4.11 |
| *Crumb hardness* | | | | |
| *after 1 day* | 514 ± 59 | 545 ± 42 | 608 ± 33 | 522 ± 64 |
| *after 2 days* | 678 ± 32 | 599 ± 61 | 725 ± 75 | 639 ± 51 |
| *after 6 days* | 873 ± 137 | 749 ± 56 | 1316 ± 45 | 895 ± 36 |

These results show that despite the fact that high temperatures were employed during the manufacture of bakery emulsions 3 and 4, hardly any enzyme activity had been lost as a result of this heat exposure, especially in case of emulsion 4.

The results also indicate that the adverse affect of heat exposure on enzyme activity may be minimized by combining the enzyme with the fat phase component, followed by addition of the aqueous phase component.

### Example 4

Starting from the recipe shown in Table 12, five bakery emulsions were produced.

**Table 12**

| | **1** | **2** | **3** | **4** | **Control** |
|---|---|---|---|---|---|
| **Fat phase** | **65%** | **65%** | **65%** | **65%** | **65%** |
| *Rapeseed oil* | 11.68% | 11.68% | 11.68% | 11.68% | 11.68% |
| *Palm oil* | 16.94% | 16.94% | 16.94% | 16.94% | 16.94% |
| *Interesterified blend* | 22.72% | 22.72% | 22.72% | 22.72% | 22.72% |
| *Palm fraction* | 13.56% | 13.56% | 13.56% | 13.56% | 13.56% |
| *Myverol 18-04¹* | 0.10% | 0.10% | 0.10% | 0.10% | 0.10% |

| **Water phase** | **35%** | **35%** | **35%** | **35%** | **35%** |
|---|---|---|---|---|---|
| *Water* | 29.95% | 29.99% | 30.00% | 30.04% | 30.20% |
| *Potassium sorbate* | 0.04% | 0.00% | 0.04% | 0.00% | 0.00% |
| *Citric acid* | 0.05% | 0.05% | 0.00% | 0.00% | 0.00% |
| *Opticake 50 BG²* | 0.16% | 0.16% | 0.16% | 0.16% | 0.00% |
| *Acid whey powder³* | 0.80% | 0.80% | 0.80% | 0.80% | 0.80% |
| *Cream (35%)* | 4.00% | 4.00% | 4.00% | 4.00% | 4.00% |
| | **100%** | **100%** | **100%** | **100%** | **100%** |

| | | | | | |
|---|---|---|---|---|---|
| ¹ ex Kerry Ingredients, distilled monoglycerides, min. 95% monoglycerides, Iodine Value max. 3, source: palm ² ex Novozymes, enzyme preparation contains maltogenic alpha-amylase (40 MAU/mg) ³ ex Prolactal | | | | | |

The bakery emulsions were prepared as follows:
- The fat blends were molten by heating to 60°C in a premix vessel.
- The emulsifier (Myverol 18-04K) was combined with part of the fat blend and dissolved therein by heating the blend to a temperature above the melting point of the emulsifier (min. 66°C). After melting the dissolved emulsifier was added to the fat blend
- The aqueous phase was prepared by successively dissolving - if required - potassium sorbate, citric acid and Opticake 50 BG into water at a temperature of 40°C. Also acid whey powder and cream were dosed.
- The aqueous phase was added to the fat phase under vigorous stirring to obtain a fat continuous emulsion. The emulsion temperature was around 65°C.
- The emulsion was heated in a plate heat exchanger (70°C / 45 seconds / intended to erase crystal memory) before being fed to a series of scraped surface heat exchangers (A-units), a crystallising unit (C-unit) and a B-unit.
- The crystallised bakery emulsion so produced was packaged in a wrapper,

Emulsions 3, 4 and control are comparative examples.

The N-line characteristics of the fat phase, the Stevens hardness and the pH of the emulsions at 20 °C are shown in Table 13.

**Table 13**

| | **1** | **2** | **3** | **4** | **Control** |
|---|---|---|---|---|---|
| *N10* | 54% | 56% | 55% | 54% | 54% |
| *N15* | 44% | 46% | 46% | 44% | 44% |
| *N20* | 35% | 37% | 36% | 35% | 34% |
| *N25* | 27% | 28% | 27% | 26% | 26% |
| *N30* | 18% | 19% | 19% | 18% | 18% |
| *N35* | 11% | 13% | 13% | 12% | 12% |
| | | | | | |
| *S20* | 254 g | 255 g | 282 g | 271 g | 292 g |
| | | | | | |
| *pH* | 4.4 | 4.3 | 5.7 | 5.6 | 5,6 |

Microbial stability was measured at regular intervals during almost 8 weeks of storage at 20°C, using SGS CTS Food Lab accredited analysis method BELAC ISO 17025, Total Aerobe Count (30°C) / g (ISO 4833) (TAC/g). The results are shown in Table 14.

**Table 14**

| **1** | | **2** | | **3** | | **4** | | **Control** | |
|---|---|---|---|---|---|---|---|---|---|
| days | TAC/g | days | TAC/g | days | TAC/g | days | TAC/g | days | TAC/g |
| 7 | 60 | 6 | 520 | 6 | 820 | 5 | 540 | 5 | <10 |
| 11 | 60 | 10 | 580 | 10 | 4400 | 9 | 800 | 9 | 30 |
| 25 | 300 | 24 | 5000 | 24 | 6.6 x 10⁶ | 23 | 6.0 x 10⁶ | 23 | 700 |
| 32 | 1800 | 31 | 1400 | 31 | 170000 | 30 | 2.9 x 10⁶ | 30 | 800 |
| 39 | 3800 | 38 | 56000 | 38 | 250000 | 37 | 1.3 x 10⁶ | 37 | 19000 |
| 46 | 600 | 45 | 70000 | 45 | 3.5 x 10⁶ | 44 | 25 x 10⁶ | 44 | 19000 |

The relatively low counts for the control sample are believed to be caused by the absence of the enzyme ingredient which according to specification had a maximum total viable count of 50 000/g.

Yeast dough (brioche) was prepared using each of the aforementioned bakery emulsions on the basis of the recipe shown in Table 15.

**Table 15**

| **Ingredient** | **Weight (g)** | **Temperature** (°C) |
|---|---|---|
| Flour | 2,000 | 20 |
| Sugar | 200 | 20 |
| Salt | 30 | 20 |
| Water | 1,000 | 5 |
| Yeast | 160 | 5 |
| Bakery emulsion | 250 | 20 |

The yeast dough was prepared as follows:
- All ingredients were kneaded in a Diosna Spiral kneader for 2 minutes at speed 1 and 3.5 minutes at speed 2. The temperature of the obtained dough was 25-26°C.
- The dough was rounded and given a resting time of 15 minutes
- The dough was divided in pieces of 600 g, the dough pieces were rounded and given a resting time of 5-10 minutes
- The dough pieces were rolled out, using a lamination table, to a final thickness of 3,5 mm in 6 steps (15mm-12.8mm-10.6mm-7mm-4.5mm-3.5mm)
- The rolled-out dough was rolled manually into a bread-like shape and placed in a greased baking tin (8 cm x 9 cm x 31 cm)
- Proofing: 40 minutes at 37°C (humidity 85%)
- Baking: 45 minutes at 200°C in deck oven

The results of the baking trials after 2 and 6 weeks storage of the emulsions are shown in Table 16.

**Table 16**

| | **1** | **2** | **3** | **4** | **Control** |
|---|---|---|---|---|---|
| **After 2 weeks** | | | | | |
| Specific volume (ml/g) | 3.67 | 3.55 | 3.67 | 3.59 | 3.70 |
| Crumb hardness | | | | | |
| After 1 day | 642 | 717 | 753 | 778 | 900 |
| After 2 days | 663 | 708 | 822 | 887 | 1211 |
| After 5 days | 934 | 769 | 1242 | 1158 | 2160 |
| After 7 days | 742 | 715 | 1027 | 1001 | 1953 |

| **After 6 weeks** | | | | | |
|---|---|---|---|---|---|
| Crumb hardness | | | | | |
| After 1 day | 680 | 639 | 599 | 624 | 787 |
| After 2 days | 711 | 782 | 659 | 665 | 1133 |
| After 5 days | 719 | 714 | 623 | 609 | 1361 |
| After 7 days | 908 | 896 | 763 | 750 | 1708 |

## Claims

1. A solid bakery emulsion comprising 40-99 wt.% of a continuous fat phase and 1-60 wt.% of a dispersed aqueous phase, said continuous fat phase having a solid fat content at 20°C (N₂₀) of at least 12%, said dispersed aqueous phase having a pH in the range of 3.2 to 5.5, and said emulsion containing an active polysaccharide hydrolysing enzyme selected from amylase, xylanase and combinations thereof.

2. Bakery emulsion according to claim 1, wherein the amylase is selected from α-amylase, maltogenic α-amylase, G4 amylase, amyloglucosidase and combinations thereof.

3. Bakery emulsion according to claim 2, wherein the emulsion contains maltogenic amylase in a concentration of 3-2400 MAU/g.

4. Bakery emulsion according to any one of the preceding claims, wherein the emulsion comprise 60-97 wt.% of continuous fat phase and 3-40 wt.% of a dispersed aqueous phase.

5. Bakery emulsion according to any one of the preceding claims, wherein the aqueous phase has a pH in the range of 3.4 to 5.3, preferably in the range of 3.5 to 5.1.

6. Bakery emulsion according to claim 5, wherein the aqueous phase contains at least 0.01 wt.% of an organic acid selected from lactic acid, citric acid, tartaric acid and combinations thereof.

7. Bakery emulsion according to any one of the preceding claims, wherein the aqueous phase contains 0.05-2 wt.% of a preservative selected from sorbate, benzoate, propionate and combinations thereof.

8. Bakery emulsion according to any one of the preceding claims, wherein the fat phase has a solid fat content at 25°C (N₂₅) of 8-30%, a solid fat content at 35°C (N₃₅) of less than 15% and a solid fat content at 40°C (N₄₀) of not more than 10%.

9. A method of preparing a dough or a batter, said method comprising thoroughly mixing flour with a bakery emulsion according to any one of the preceding claims.

10. Method according to claim 9, wherein the bakery emulsion is employed in a concentration of 5-110% by weight of flour.

11. Method according to claim 9 or 10, wherein the flour is combined with the bakery emulsion, sugar, eggs and further bakery ingredient to prepare a cake batter.

12. Method according to claim 9 or 10, wherein the flour is combined with the bakery emulsion, active yeast, aqueous liquid and optionally further bakery ingredients to prepare a yeast-leavened dough.

13. A process of producing a bakery emulsion according to any one of claims 1-8, said process comprising:
• preparing an aqueous phase composition containing the polysaccharide hydrolysing enzyme and maintaining the temperature of the aqueous phase composition below 85°C;
• preparing a fat phase composition;
• mixing the aqueous phase composition and the fat phase composition to prepare a pre-emulsion, said pre-emulsion having a temperature in the range of 40-85 °C; and
• cooling the pre-emulsion.

14. A process of producing a bakery emulsion according to any one of claims 1- 8, said process comprising:
• preparing an aqueous phase composition;
• preparing a fat phase composition containing the polysaccharide hydrolysing enzyme and maintaining the temperature of the fat phase composition below 85°C;
• mixing the aqueous phase composition and the fat phase composition to prepare a pre-emulsion, said pre-emulsion having a temperature in the range of 40-85 °C; and
• cooling the pre-emulsion.

15. Process according to claim 13 or 14, wherein the pre-emulsion is kept at a temperature of at least 50°C for at least 15 seconds before the cooling in a scraped surface heat exchanger.

## Patentansprüche

1. Feste Backemulsion umfassend 40-99 Gew.-% einer kontinuierlichen Fettphase und 1-60 Gew.-% einer dispergierten wässrigen Phase, wobei die kontinuierliche Fettphase einen Festfettgehalt bei 20 °C (N₂₀) von wenigstens 12 % aufweist, die dispergierte wässrige Phase einen pH im Bereich von 3,2 bis 5,5 aufweist, und die Emulsion ein aktives Polysaccharid-hydrolysierendes Enzym, ausgewählt aus Amylase, Xylanase und Kombinationen davon, enthält.

2. Backemulsion gemäß Anspruch 1, wobei die Amylase aus α-Amylase, maltogener α-Amylase, G4-Amylase, Amyloglucosidase und Kombinationen davon ausgewählt ist.

3. Backemulsion gemäß Anspruch 2, wobei die Emulsion maltogene Amylase in einer Konzentration von 3-2400 MAU/g enthält.

4. Backemulsion gemäß einem der vorangehenden Ansprüche, wobei die Emulsion 60-97 Gew.-% kontinuierliche Fettphase und 3-40 Gew.-% einer dispergierten wässrigen Phase umfasst.

5. Backemulsion gemäß einem der vorangehenden Ansprüche, wobei die wässrige Phase einen pH im Bereich von 3,4 bis 5,3, vorzugsweise im Bereich von 3,5 bis 5,1 aufweist.

6. Backemulsion gemäß Anspruch 5, wobei die wässrige Phase wenigstens 0,01 Gew.-% einer organischen Säure, ausgewählt aus Milchsäure, Citronensäure, Weinsäure und Kombinationen davon, enthält.

7. Backemulsion gemäß einem der vorangehenden Ansprüche, wobei die wässrige Phase 0,05-2 Gew.-% eines Konservierungsmittels, ausgewählt aus Sorbat, Benzoat, Propionat und Kombinationen davon, enthält.

8. Backemulsion gemäß einem der vorangehenden Ansprüche, wobei die Fettphase einen Festfettgehalt bei 25 °C (N₂₅) von 8-30 %, einen Festfettgehalt bei 35 °C (N₃₅) von weniger als 15 % und einen Festfettgehalt bei 40 °C (N₄₀) von nicht mehr als 10 % aufweist.

9. Verfahren zum Herstellen eines Teigs oder eines Rührteigs, wobei das Verfahren das gründliche Mischen von Mehl mit einer Backemulsion gemäß einem der vorangehenden Ansprüche umfasst.

10. Verfahren gemäß Anspruch 9, wobei die Backemulsion in einer Konzentration von 5-110 Gew.-% des Mehls eingesetzt wird.

11. Verfahren gemäß Anspruch 9 oder 10, wobei das Mehl mit der Backemulsion, Zucker, Eiern und weiteren Backzutaten vereinigt wird, um einen Kuchenteig herzustellen.

12. Verfahren gemäß Anspruch 9 oder 10, wobei das Mehl mit der Backemulsion, aktiver Hefe, wässriger Flüssigkeit und gegebenenfalls weiteren Backzutaten vereinigt wird, um einen durch Hefe gelockerten Teig herzustellen.

13. Verfahren zum Herstellen einer Backemulsion gemäß einem der Ansprüche 1-8, wobei das Verfahren umfasst:
• Herstellen einer Zusammensetzung der wässrigen Phase, die das Polysaccharid-hydrolysierende Enzym enthält, und Halten der Temperatur der Zusammensetzung der wässrigen Phase unter 85 °C;
• Herstellen einer Zusammensetzung der Fettphase;
• Mischen der Zusammensetzung der wässrigen Phase und der Zusammensetzung der Fettphase, um eine Voremulsion herzustellen, wobei die Voremulsion eine Temperatur im Bereich von 40-85 °C aufweist; und
• Kühlen der Voremulsion.

14. Verfahren zum Herstellen einer Backemulsion gemäß einem der Ansprüche 1-8, wobei das Verfahren umfasst:
• Herstellen einer Zusammensetzung der wässrigen Phase;
• Herstellen einer Zusammensetzung der Fettphase, die das Polysaccharid-hydrolysierende Enzym enthält, und Halten der Temperatur der Zusammensetzung der Fettphase unter 85 °C;
• Mischen der Zusammensetzung der wässrigen Phase und der Zusammensetzung der Fettphase, um eine Voremulsion herzustellen, wobei die Voremulsion eine Temperatur im Bereich von 40-85 °C aufweist; und
• Kühlen der Voremulsion.

15. Verfahren gemäß Anspruch 13 oder 14, wobei die Voremulsion vor dem Kühlen in einem Kratzwärmetauscher wenigstens 15 Sekunden auf einer Temperatur von wenigstens 50 °C gehalten wird.

## Revendications

1. Emulsion de boulangerie solide comprenant 40 à 99 % en poids d'une phase grasse continue et 1 à 60 % en poids d'une phase aqueuse dispersée, ladite phase grasse continue ayant une teneur en matière grasse solide à 20°C (N₂₀) d'au moins 12 %, ladite phase aqueuse dispersée ayant un pH dans la plage de 3,2 à 5,5, et ladite émulsion contenant une enzyme active hydrolysant les polysaccharides choisie parmi l'amylase, la xylanase et des combinaisons de celles-ci.

2. Emulsion de boulangerie selon la revendication 1, dans laquelle l'amylase est choisie parmi l'a-amylase, l'a-amylase maltogène, l'amylase G4, l'amyloglucosidase et des combinaisons de celles-ci.

3. Emulsion de boulangerie selon la revendication 2, dans laquelle l'émulsion contient de l'amylase maltogène à une concentration de 3 à 2 400 MAU/g.

4. Emulsion de boulangerie selon l'une quelconque des revendications précédentes, dans laquelle l'émulsion comprend 60 à 97 % en poids de phase grasse continue et 3 à 40 % en poids d'une phase aqueuse dispersée.

5. Emulsion de boulangerie selon l'une quelconque des revendications précédentes, dans laquelle la phase aqueuse a un pH dans la plage de 3,4 à 5,3, de préférence dans la plage de 3,5 à 5,1.

6. Emulsion de boulangerie selon la revendication 5, dans laquelle la phase aqueuse contient au moins 0,01 % en poids d'un acide organique choisi parmi l'acide lactique, l'acide citrique, l'acide tartrique et des combinaisons de ceux-ci.

7. Emulsion de boulangerie selon l'une quelconque des revendications précédentes, dans laquelle la phase aqueuse contient 0,05 à 2 % en poids d'un conservateur choisi parmi le sorbate, le benzoate, le propionate et des combinaisons de ceux-ci.

8. Emulsion de boulangerie selon l'une quelconque des revendications précédentes, dans laquelle la phase grasse a une teneur en matière grasse solide à 25°C (N₂₅) de 8 à 30 %, une teneur en matière grasse solide à 35°C (N₃₅) inférieure à 15 % et une teneur en graisse solide à 40°C (N₄₀) de pas plus de 10 %.

9. Procédé de préparation d'un pâton ou d'une pâte, ledit procédé consistant à mélanger intimement la farine avec une émulsion de boulangerie selon l'une quelconque des revendications précédentes.

10. Procédé selon la revendication 9, dans lequel l'émulsion de boulangerie est utilisée à une concentration de 5 à 110 % en poids de farine.

11. Procédé selon la revendication 9 ou 10, dans lequel la farine est combinée avec l'émulsion de boulangerie, du sucre, des oeufs et un ingrédient de boulangerie supplémentaire pour préparer une pâte à gâteau.

12. Procédé selon la revendication 9 ou 10, dans lequel la farine est combinée avec l'émulsion de boulangerie, de la levure active, un liquide aqueux et facultativement des ingrédients de boulangerie supplémentaires pour préparer une pâte levée à la levure.

13. Procédé de production d'une émulsion de boulangerie selon l'une quelconque des revendications 1 à 8, ledit procédé comprenant les étapes consistant à :
• préparer une composition en phase aqueuse contenant l'enzyme hydrolysant les polysaccharides et maintenir la température de la composition en phase aqueuse en dessous de 85°C ;
• préparer une composition en phase grasse ;
• mélanger la composition en phase aqueuse et de la composition en phase grasse pour préparer une pré-émulsion, ladite pré-émulsion ayant une température comprise entre 40 et 85°C ; et
• refroidir la pré-émulsion.

14. Procédé de production d'une émulsion de boulangerie selon l'une quelconque des revendications 1 à 8, ledit procédé comprenant les étapes consistant à :
• préparer une composition en phase aqueuse ;
• préparer une composition en phase grasse contenant l'enzyme hydrolysant les polysaccharides et maintenir la température de la composition en phase grasse en dessous de 85°C ;
• mélanger la composition en phase aqueuse et de la composition en phase grasse pour préparer une pré-émulsion, ladite pré-émulsion ayant une température comprise entre 40 et 85°C ; et
• refroidir la pré-émulsion.

15. Procédé selon la revendication 13 ou 14, dans lequel la pré-émulsion est maintenue à une température d'au moins 50°C pendant au moins 15 secondes avant le refroidissement dans un échangeur de chaleur à surface raclée.
